# EUROPEAN PATENT SPECIFICATION

(11) **EP 3 505 170 B1**
(45) Date of publication and mention of the grant of the patent: **11.08.2021**
(21) Application number: 17842945.2
(22) Date of filing: 23.08.2017
(51) Int. Cl.: A61K 31/4174, A61K 9/08, A61P 27/12

(54) **AZOLE COMPOUND OPHTHALMIC PREPARATION**
OPHTHALMISCHES AZOLVERBINDUNGSPRÄPARAT
PRÉPARATION OPHTALMIQUE COMPRENANT UN COMPOSÉ AZOLE

(30) Priority: 24.08.2016 CN 201610717956
(43) Date of publication of application: 03.07.2019
(73) Proprietor: Biotool, LLC, Shanghai 200120 (CN); Zheng, Qinyuan, Shanghai 200020 (CN); INHIBIO BIOSCIENCE PTE. LTD., Singapore 068809 (SG)
(72) Inventor: ZHENG, Qinyuan, Shanghai 200020 (CN); JIAPAER, Zeyidan, Shanghai 200131 (CN)
(74) Representative: Predazzi, Valentina
(86) International application number: PCT/CN2017/098662
(87) International publication number: WO 2018/036523

(56) References cited:
- WO-A1-2016/029199
- CN-A- 101 766 628
- CN-A- 102 008 428
- CN-A- 103 736 093
- ABD EL-GAWAD ABD EL-GAWAD ET AL: "Improvement of the Ocular Bioavailability of Econazole Nitrate upon Complexation with Cyclodextrins", AAPS PHARMSCITECH, SPRINGER US, NEW YORK, vol. 18, no. 5, 9 November 2016 (2016-11-09), pages 1795-1809, XP036266252, DOI: 10.1208/S12249-016-0609-9 [retrieved on 2016-11-09]
- AMR MAGED ET AL: "Nano Spray Drying Technique as a Novel Approach To Formulate Stable Econazole Nitrate Nanosuspension Formulations for Ocular Use", MOLECULAR PHARMACEUTICS, vol. 13, no. 9, 31 March 2016 (2016-03-31) , pages 2951-2965, XP055677316, US ISSN: 1543-8384, DOI: 10.1021/acs.molpharmaceut.6b00167
- AZZA A MAHMOUD ET AL: "Chitosan/sulfobutylether--cyclodextrin nanoparticles as a potential approach for ocular drug delivery", INTERNATIONAL JOURNAL OF PHARMACEUTICS, ELSEVIER, NL, vol. 413, no. 1, 14 April 2011 (2011-04-14), pages 229-236, XP028374015, ISSN: 0378-5173, DOI: 10.1016/J.IJPHARM.2011.04.031 [retrieved on 2011-04-21]
- LIN CHUAN-YI SU ET AL: "Intraocular use of fluconazole in the management of ocular fungal infection", KAOHSIUNG JOURNAL OF MEDICAL SCIENCES, vol. 15, no. 4, 1 April 1999 (1999-04-01), pages 218-225, XP055677265,

## Description

### Field of Invention

The present invention relates to the field of ocular medicines, in particular, the invention discloses an ophthalmic preparation form of an econazole compound, and the use thereof for treating or preventing ocular lens diseases such as cataract.

### Background of Invention

Cataract is a common blinding eye disease with blurred vision and vision loss as the main symptoms. Among the 40 million to 45 million blind people in the world, 60% of the blindness are due to cataract. Cataract occurs in the ocular lens. Due to aging, genetics, local dystrophies, immunity and metabolic abnormalities, trauma, radiation and other factors, a sick individual's lens metabolism is disordered, resulting in the denaturation and error accumulation of lens protein, thereby affecting the amount of light entering the eye and reaching the retina. The eventual result is a symptom of blurred vision or even complete loss of vision (Bloemendal, de Jong et al. 2004).

The incidence of cataract is not limited to humans, and many mammalian species (horses, dogs, monkeys, etc.) can develop cataracts (Chauke, Magwebu et al. 2016; Sande, Alvarez et al. 2016). Cataracts can be classified into senile cataracts, congenital cataracts, traumatic cataracts, and concurrent cataracts according to different causes. At present, there is no clinical drug that can effectively treat cataract, and the sick individual can only improve the vision by replacing with artificial lens by surgery.

An azole compound is from a class of five-membered heterocyclic compounds containing one nitrogen atom and at least one non-carbon atom. Due to the human acceptable toxicity, azole compounds have been always used in mammalian antifungal treatments. As early as the 1980s, ketoconazole was used for oral treatment of systemic fungal infections. With the discovery and synthesis of more azole compounds, their own antibacterial spectrum is more extensive, and the corresponding pharmacokinetic properties and safety are more suitable for clinical use (Maertens JA, 2004). To date, FDA has approved many azole compounds for clinical antifungal use (http://www.fda.gov/default.htm).

This following document is published after the effective filling date of present application: ABD EL-GAWAD ABD EL-GAWAD ET AL: "Improvement of the Ocular Bioavailability of Econazole Nitrate upon Complexation with Cyclodextrins", AAPS PHARMSCITECH, vol. 18, no. 5, 9 November 2016 (2016-11-09), pages 1795-1809.

WO 2016/029199 A1 describes the treatment of cataract using lanesterol.

These following documents describe econazolecompositions, but fail to describe the treatment of cataract:
- AMR MAGED ET AL: "Nano Spray Drying Technique as a Novel Approach To Formulate Stable Econazole Nitrate Nanosuspension Formulations for Ocular Use", MOLECULAR PHARMACEUTICS, vol. 13, no. 9, 31 March 2016 (2016-03-31), pages 2951-2965;
- AZZA A MAHMOUD ET AL: "Chitosan/sulfobutylether--cyclodextrin nanoparticles as a potential approach for ocular drug delivery", INTERNATIONAL JOURNAL OF PHARMACEUTICS, ELSEVIER, NL, vol. 413, no. 1, 14 April 2011 (2011-04-14), pages 229-236;
- LIN CHUAN-YI SU ET AL: "Intraocular use of fluconazole in the management of ocular fungal infection", KAOHSIUNG JOURNAL OF MEDICAL SCIENCES, vol. 15, no. 4, 1 April 1999 (1999-04-01), pages 218-225.

The present invention is the first to confirm the effect of an azole compound, being an econazole compound, in the treatment and prevention of ocular lens diseases such as ocular cataract in mammals.

### Summary of Invention

The purpose of the invention is to provide an econazole compound ophthalmic preparation with high concentration, suitable osmotic pressure, good eye tolerance, and is suitable for ocular administration.

Another purpose of the invention is to provide use of the preparation (especially an ophthalmic preparation) for treating or preventing cataract in human or non-human mammals.

In the first aspect of the invention, it provides a non-invasive administration ophthalmic preparation, which comprises: (a) a pharmaceutically acceptable carrier, and (b) an azole compound, being an econazole compound as a first active ingredient; wherein the concentration of the azole compound in the ophthalmic preparation is from 0.005 to 400 µM.

The azole compound is an econazole compound comprising econazole, pharmaceutically acceptable salt thereof, and combinations thereof.

In another preferred embodiment, in the ophthalmic preparation, all or substantially all of the azole compound is dissolved.

In another preferred embodiment, the term "all or substantially all" means 90-100%, preferably 95-100%, more preferably 99-100%.

In another preferred embodiment, in the ophthalmic preparation, the concentration of the azole compound is from 0.01 to 200 µM, preferably from 0.025 to 100 µM, more preferably from 0.05 to 50 µM; most preferably from 0.5 to 10 µM.

In another preferred embodiment, the concentration of the azole compound is from about 5 to 8 µM.

In another preferred embodiment, the concentration of the azole compound is 7 µM.

In another preferred embodiment, the azole compound is econazole.

In another preferred embodiment, the ophthalmic preparation is selected from the group consisting of: eye drop, an emulsion, gel, eye ointment, sustained-release microsphere, intraocular sustained-release graft, and ocular sustained-release drug film.

In another preferred embodiment, the eye drops are solution.

In another preferred embodiment, the eye drops are emulsion.

In another preferred embodiment, the ophthalmic preparation is a homogeneous solution.

In another preferred embodiment, the ophthalmic formulation further comprises a solid pharmaceutical formulation which can be reconstructed into a liquid (i.e. the formulation can be directly reconstructed into a liquid ophthalmic preparation after adding a liquid pharmaceutically acceptable carrier).

In another preferred embodiment, the liquid pharmaceutically acceptable carrier is water.

In another preferred embodiment, the ophthalmic preparation further comprises: (c) a second active ingredient, wherein the second active ingredient is selected from the group consisting of: steroid compound, glucocorticoid compound, antibiotics, and combinations thereof.

In another preferred embodiment, the second active ingredient is lanosterol compound.

In another preferred embodiment, the concentration of the lanosterol compound is from 10 to 200 mM, preferably from 15 to 150 mM, more preferably from 20 to 50 mM; most preferably from 20 to 30 mM.

In another preferred embodiment, the lanosterol compound is selected from the group consisting of:
(i) lanosterol, or a pharmaceutically acceptable salt thereof, or a pharmaceutically acceptable ester thereof;
(ii) dihydro lanosterol, or a pharmaceutically acceptable salt thereof, or a pharmaceutically acceptable ester thereof; and
(iii) a combination of the above components (i) and (ii).

In another preferred embodiment, the lanosterol compound is lanosterol.

In another preferred embodiment, the lanosterol compound is dihydro lanosterol.

In another preferred embodiment, the lanosterol compound is a mixture of lanosterol and dihydro lanosterol.

In another preferred embodiment, in the mixture of lanosterol and dihydro lanosterol, the ratio of the lanosterol content C1 to the dihydro lanosterol content C2 (C1/C2) is from 1:500 to 500:1, preferably from 5:90 to 500:1, more preferably from 80:1 to 200:1, most preferably from 85:1 to 100:1.

In another preferred embodiment, the glucocorticoid compound is selected from the group consisting of: dexamethasone, hydrocortisone, and combinations thereof.

In another preferred embodiment, the antibiotic is selected from the group consisting of: tobramycin, gentamicin sulfate, chlortetracycline, chloramphenicol, and combinations thereof.

In another preferred embodiment, in the ophthalmic preparation, the second active ingredient is in a dissolved form.

In another preferred embodiment, in the ophthalmic preparation, the content of the second active ingredient is from 0.01 to 15wt%, preferably from 0.5 to 3wt%, based on the total weight of the ophthalmic formulation.

In another preferred embodiment, the pharmaceutically acceptable carrier is non-irritating to the eye.

In another preferred embodiment, the pharmaceutically acceptable carrier comprises one or more carriers selected from the group consisting of:
(a1) water;
(a2) a solubilizer;
(a3) a surfactant;
(a4) a thickener;
(a5) an osmotic pressure adjusting agent;
(a6) a buffer or a buffer solution composed of the buffer;
(a7) a preservative;
(a8) a chelating agent;
(a9) a sustained-release agent.

In another preferred embodiment, the solubilizer comprises a polyhydroxy compound.

In another preferred embodiment, the ratio of the amount of the polyhydroxy compound to the first active ingredient is 1:40000-1:1.

In another preferred embodiment, the polyhydroxy compound is selected from: polyhydroxy alcohol, cyclodextrins, polyvinyl alcohol, and combinations thereof.

In another preferred embodiment, the polyhydroxy compounds have a skeleton composed of carbon atoms, hydrogen atoms, and heteroatoms such as N, and the reactive group are substantially or completely hydroxyl.

In another preferred embodiment, the polyhydroxy compounds includes alcohol polyhydroxy compounds (such as C2-C10 polyol), and cyclodextrins and cyclodextrin derivatives.

In another preferred embodiment, the polyhydroxy compound is selected from: polyene glycol, glycerol, polyethylene glycol, α-cyclodextrin, β-cyclodextrin, γ-cyclodextrin, cyclodextrin derivatives, polyvinyl alcohol (PVA), and any combination thereof.

In another preferred embodiment, the polyhydroxy compound is hydroxypropyl-β-cyclodextrin.

In another preferred embodiment, the surfactant is selected from: anionic surfactants, cationic surfactants, nonionic surfactants, chaotropic surfactants, and any combination thereof.

In another preferred embodiment, the nonionic surfactant is selected from: Tween, Span, fatty acid glyceryl esters, polyoxyethylenes, polyoxyethylene-polyoxypropylene copolymers, and any combination thereof.

In another preferred embodiment, the thickener is selected from: chitosan, hydroxypropyl methylcellulose (HPMC), methylcellulose (MC), povidone (PVP), gelatin, sodium carboxymethylcellulose (CMC-Na), and any combination thereof.

In another preferred embodiment, the thickener is chitosan.

In another preferred embodiment, the osmotic pressure adjusting agent is selected from: carbohydrate compound, salt compound, and any combination thereof.

In another preferred embodiment, the carbohydrate compound is selected from: sorbitol, glucose, mannitol, and any combination thereof.

In another preferred embodiment, the salt compound is selected from: sodium chloride, potassium chloride, boric acid, and any combination thereof.

In another preferred embodiment, the buffer solution is selected from: phosphate buffer, borate buffer, citrate buffer, tartaric acid buffer, ammonium acetate buffer, and any combination thereof.

In another preferred embodiment, the preservative is selected from: a benzalkonium bromide, a chlorobutanol, a p-hydroxybenzoate, a sorbic acid, an antibiotic, and any combination thereof; preferably, the content of preservative is 0-1wt%.

In another preferred embodiment, the ophthalmic preparation contains no preservative.

In another preferred embodiment, the chelating agent is selected from: EDTA, EGTA, CDTA, a citrate, and any combination thereof; preferably, the content of chelating agent is 0-0.1 wt%.

In another preferred embodiment, the ophthalmic preparation contains a polyhydroxy compound, optionally a surfactant and optionally a thickener,
wherein, the content of the polyhydroxy compound is 0.1-50wt%;
the content of the surfactant is 0-2wt%;
the content of the thickener is 0-6wt%;
based on the total weight of the ophthalmic preparation.

In another preferred embodiment, the content of the polyhydroxy compound is 25-40wt%.

In another preferred embodiment, the content of the surfactant is 0.1-1wt%.

In another preferred embodiment, the content of the thickener is 0.1-5wt%.

In another preferred embodiment, the osmotic pressure of the ophthalmic preparation is 240-5 10mOsm.

In another preferred embodiment, the pH value of the ophthalmic preparation is from 5.5 to 8.5, preferably from 6.0 to 8.0, and more preferably from 6.5 to 7.5.

In another preferred embodiment, the ophthalmic preparation is an aqueous solution for ocular administration.

In another preferred embodiment, the ophthalmic preparation is an aqueous solution for ocular administration, and the concentration of the lanosterol compound in the solution is from 10 to 200 mM, preferably from 15 to 150 mM, more preferably from 20 to 50 mM; and most preferably from 20 to 30 mM.

In another preferred embodiment, the ophthalmic preparation contains the following ingredients:
0.05-40µM econazole compound;
10-50mM lanosterol compound;
0.1-50wt% polyhydroxy compound; preferably propylene glycol or β cyclodextrin;
0-1wt% solubilizer, preferably polysorbate;
0.2-0.4wt% thickener, preferably chitosan;
0-0.5wt% preservative, preferably chloramphenicol;
and the balance of water,
and the ophthalmic preparation has a pH of about 6.5-7.5 and an osmotic pressure of 240-510 mOsm.

In the second aspect of the invention, it provides a method for preparing the ophthalmic preparation provided in the first aspect of the present invention, which comprises the following step:
(1) mixing (a) a pharmaceutically acceptable carrier and (b) an econazole compound as a first active ingredient, thereby forming the ophthalmic preparation provided in the first aspect of the present invention.

In another preferred embodiment, the step (1) comprises mixing (a) a pharmaceutically acceptable carrier and (b) an econazole compound as a first active ingredient and (c) the second active ingredient, thereby forming the ophthalmic preparation provided in the first aspect of the present invention.

In another preferred embodiment, the method comprises:
(i) dispersing an econazole compound as a first active ingredient and optionally a second active ingredient in a polyhydroxy compound to form a first dispersion;
(ii) mixing the first dispersion with the remaining pharmaceutically acceptable carrier, thereby forming the ophthalmic preparation provided in the first aspect of the present invention.

In another preferred embodiment, the step (ii) comprises firstly mixing the remaining pharmaceutically acceptable carrier to form a second solution or a second dispersion, and then mixing the first dispersion with the second solution or the second dispersion, thereby forming the ophthalmic preparation of the first aspect of the invention.

In another preferred embodiment, the solvent of the second solution is water.

In another preferred embodiment, the solute of the second solution is selected from the group consisting of: a solubilizer, a surfactant, a thickener, an osmotic pressure adjusting agents, a buffer, a preservative, a chelating agent, a sustained-release agent, and combinations thereof.

In the third aspect of the invention, a use of the ophthalmic preparation of the first aspect of the invention is provided, wherein the ophthalmic preparation is used for preparation of a drug for preventing or treating human or non-human mammal ocular lens diseases (such as cataract, presbyopia, or a combination thereof).

In another preferred embodiment, the non-human mammal comprises horse, dog, cat, panda, monkey, orangutan, rodent, rabbit, elephant, tiger.

In another preferred embodiment, the rodents include mice and rats.

In another preferred embodiment, the cataract is selected from the group consisting of: senile cataract, congenital cataract, traumatic cataract, and concurrent cataract.

In another preferred embodiment, the cataract is traumatic cataract.

In another preferred embodiment, the presbyopic patient is more than 48 years old (preferably ≥60 years old).

In the fourth aspect of the invention, it provides a method for preventing or treating ocular lens diseases such as cataract, comprising: administering the ophthalmic preparation of the first aspect of the invention non-invasively to the eye(s) of a subject in need thereof.

In another preferred embodiment, the ocular lens disease is selected from the group consisting of: cataract, presbyopia, and combinations thereof.

In another preferred embodiment, the "administering non-invasively" means dripping into the eye.

Further, the present invention also provides a method for preventing or treating ocular lens diseases such as cataract, which comprises: orally administering the oral preparation of the present invention or administering the injection formulation of the present invention to the eye(s) of a subject in need thereof.

In another preferred embodiment, the oral preparation or injection formulation comprises (a1) a pharmaceutically acceptable carrier, and (b1) the above-mentioned azole compound as a first active ingredient; wherein the concentration of the azole compound in the preparation is 0.01-90wt%.

### DESCRIPTION OF THE DRAWINGS

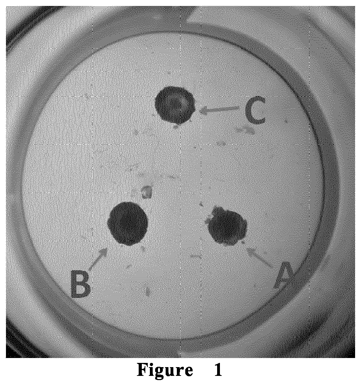

Figure 1 shows the therapeutic effect of the ophthalmic preparation in one embodiment of the present invention. The results showed that the therapeutic effect on cataractous eyes of rats of the pharmacy group containing the azole compound econazole and lanosterol was far superior to the only-lanosterol pharmacy group and the PBS solvent control group.

### DETAILED DESCRIPTION OF THE INVENTION

Through extensive and intensive researches, the inventors have unexpectedly discovered that a non-invasive administrating ophthalmic preparation containing a specific compound (i.e., an econazole compound) as an active ingredient can extremely effectively alleviate cataract symptoms in mammals, or even completely eliminate cataract symptoms in mammals. In addition, by adding a specific pharmaceutically acceptable carrier (especially a polyhydroxy compound) and an additional active ingredient (such as a lanosterol compound), it is possible to produce an ophthalmic preparation which is less irritating, has high eye tolerance, has a longer residence time of the drug in the eye, and is more effective. On the basis of the above, the inventors have completed the present invention.

### Ophthalmic preparation

An ophthalmic preparation is provided in the present invention, by selecting suitable pharmaceutical compositions (such as lanosterol compounds, polyhydroxy compounds, surfactants, thickeners, etc.), it not only successfully satisfies the special requirements of ocular medications (e.g. osmotic pressure) but also significantly improves or increases the concentration of the active ingredient.

The ophthalmic preparation of the present invention comprises a pharmaceutically acceptable carrier and an effective amount of an econazole compound as an active ingredient, and the concentration of the dissolved (i.e., free) azole compound is from 0.005 to 400 µM based on the total volume of the preparation.

In general, the ophthalmic preparation of the present invention comprises water or an aqueous solvent together with the active ingredient dissolved in the solvent and the following components: lanosterol compounds, polyhydroxy compounds, optional surfactants and optionally thickeners. The ophthalmic preparation may also optionally comprise other pharmaceutically acceptable components, and the other pharmaceutically acceptable components include but are not limited to: osmotic pressure adjusting agents, buffering agents, preservatives, chelating agents, sustained-release agents, etc.

### First active ingredient

As used herein, the terms "first active ingredient" or "azole compound" are used interchangeably and refer to an econazole compound. The active ingredient of the present invention may be various crystal forms, amorphous substance, anhydrate, solvate, hydrate, enantiomer of pharmaceutically acceptable econazole compounds. The econazole compound in the present invention means the first active ingredient of the present invention.

Econazole is a five-membered heterocyclic compound and the structure is shown as follows:

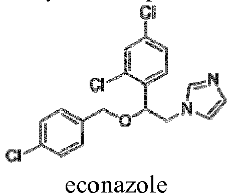

The research of the inventors has shown that, by using a specific formulation, econazole can has a very effective therapeutic effect on cataract while a high concentration thereof in the eye is maintained.

### Second active ingredient

As used herein, the term "second active ingredient" refers to a steroid compound, a glucocorticoid compound, an antibiotic, and any combination thereof. In the ophthalmic preparation of the present invention, the second active ingredient may be in a dissolved form. In the ophthalmic preparation of the present invention, the second active ingredient is present in an amount of 0.01-20wt%, preferably of 5-15wt%, based on the total weight of the ophthalmic preparation.

When the "second active ingredient" of the present invention is a lanosterol compound, the concentration thereof is preferably from 10 to 200 mM, preferably from 15 to 150 mM, more preferably from 20 to 50 mM; most preferably from 20 to 30 mM.

The above steroid compound is selected from:
(i) lanosterol, or a pharmaceutically acceptable salt thereof, or a pharmaceutically acceptable ester thereof;
(ii) dihydro lanosterol, or a pharmaceutically acceptable salt thereof, or a pharmaceutically acceptable ester thereof;
(iii) 25-hydroxycholesterol, or a pharmaceutically acceptable salt thereof, or a pharmaceutically acceptable ester thereof;
(iiii) any combination of the above components (i) and/or (ii) and/or (iii).

The glucocorticoid compound of the present invention is selected from the group consisting of: dexamethasone, hydrocortisone, and combinations thereof.

The antibiotic of the present invention is selected from the group consisting of: tobramycin, gentamicin sulfate, chlortetracycline, chloramphenicol, and combinations thereof.

### Polyhydroxy compounds

As used herein, the term "polyhydroxy compound" refers to a compound having two or more hydroxyl groups in the molecule. The inventors of the present invention have unexpectedly discovered that when the polyhydroxy compound is used in combination with an azole compound, on the one hand, the water solubility of the azole compound and the stability of the drug can be improved, and on the other hand, it does not adversely affect the azole compound. In addition, it also helps to increase the residence time of the first active ingredient in the eye, thereby further improving the efficacy of treating cataract.

The polyhydroxy compound preferably has a skeleton composed of carbon atoms, hydrogen atoms and hetero atoms (e.g. N), and the reactive group is substantially or wholly hydroxyl groups.

In another preferred embodiment, the polyhydroxy compound includes alcohol polyhydroxy compounds (such as a C2-C10 polyol), and cyclodextrins and cyclodextrin derivatives.

In another preferred embodiment, the polyhydroxy compound is selected from the group consisting of: propylene glycols, glycerols, polyethylene glycols, modified or unmodified cyclodextrins and derivatives thereof, and combinations thereof.

In another preferred embodiment, the polyhydroxy compound is hydroxypropyl-β-cyclodextrin.

The amount of polyhydroxy compounds may vary depending on the form and the usage of the preparation, and the type of the compound. In the present invention, the amount (or content) of the polyhydroxy compound in the aqueous solution of azole compounds is generally 0.1-50wt%. For example, in the present invention, 1-15wt% propylene glycol, or 20-50wt% cyclodextrin may be used.

### Other pharmaceutically acceptable carrier

In the present invention, in addition to polyhydroxy compounds, the ophthalmic preparation may further contain other pharmaceutically acceptable carriers. Representative examples includes, but not limited to, surfactants, thickeners, osmotic pressure adjusting agents, buffer agents, preservatives, chelating agents, sustained-release agents.

### Surfactant

In the present invention, the surfactant is selected from: anionic surfactants, cationic surfactants, nonionic surfactants, chaotropic surfactants, and any combination thereof. Wherein the nonionic surfactant is selected from: Tween, Span, fatty acid glycerides, polyoxyethylenes, polyoxyethylene-polyoxypropylene copolymers, and any combination thereof. Generally, the surfactant is used in an amount (or content) of 0-2wt%, more preferably 0.1-1wt %.

### Thickener

Thickeners can be used to increase the viscosity of the system and make the system maintain a uniform, stable suspension or emulsion state. The present invention increases the retention time of the drug in the eye by adding an appropriate amount of thickener, thereby increasing the absorption of the effective ingredient azole compound in the eye.

In the present invention, the thickener is preferably chitosan, hydroxypropylmethylcellulose (HPMC), methylcellulose (MC), and povidone (PVP), gelatin, sodium carboxymethylcellulose (CMC)-Na) and so on.

Generally, the thickener is used in an amount (or content) of 0-6 wt%, preferably 0.1-5 wt%.

In addition, the ophthalmic preparation of the present invention may also contain additional pharmaceutically acceptable carriers which include (but are not limited to): osmotic pressure adjusting agents, buffering agents, preservatives, chelating agents, sustained-release agents.

For example, adding a certain amount of chelating agents, such as EDTA, can increase the stability of the preparation. Generally, the concentration of the chelating agent ranges from 0 to 0.05% by weight.

In general, the type and amount of the additional pharmaceutically acceptable carrier are not particularly limited as long as the dissolution or therapeutic activity of the active ingredient is not affected.

Generally, these other pharmaceutically acceptable carriers are present in a total amount of 0.1-80wt%, preferably 1-50wt%.

### Preparation of ophthalmic preparation

The ophthalmic preparation of the present invention can be prepared by conventional equipments and methods in accordance with the pharmaceutical compositions and ratios provided in the methods of the present invention. It includes the following methods:
method 1: mixing (a) a pharmaceutically acceptable carrier; and (b) an econazole compound as a first active ingredient, thereby forming the ophthalmic preparation of the present invention.
method 2: mixing (a) a pharmaceutically acceptable carrier; and (b) an econazole compound as a first active ingredient and (c) the second active ingredient, thereby forming the ophthalmic preparation of the present invention.
method 3:
   (i) dispersing an econazole compound as a first active ingredient and optionally a second active ingredient in a polyhydroxy compound to form a first dispersion;
   (ii) mixing the first dispersion with the remaining pharmaceutically acceptable carrier to form the ophthalmic preparation of the present invention.

In step (ii), it is also possible to mix the remaining pharmaceutically acceptable carrier first to form a second solution or a second dispersion, and then mix the first dispersion with the second solution or the second dispersion, thereby forming the ophthalmic preparation of the present invention.

In the case of eye drops, it can be prepared according to any of the above three methods, followed by adjusting the pH, sterilization and filling it into a suitable container.

An aqueous solution for ocular administration prepared as described above can be used for topical administration to the eye.

### Use

The ophthalmic preparation of the present invention can be used for the preventing or treating ocular lens diseases of human or non-human mammals, such as cataracts and presbyopia etc. Representative cataracts are selected from: senile cataracts, congenital cataracts, traumatic cataracts, and concurrent cataracts.

In another preferred embodiment, the non-human mammal includes (but is not limited to) pets (such as dogs, cats), livestock (such as cattle, sheep, horses, pigs), various zoo animals (pandas, elephants).

The usage and amount of the preparation are not limited, and may be adjusted depending on the condition of the patient and the type of cataract. The above adjustments can be derived by those skilled in the art by combining the symptoms of the patient with prior arts and common knowledge in the art.

### The main advantages of the invention are:

1) It can be directly applied to the eye, and is easy to administer and has good tolerance, and has a long residence time in the eye, and has good curative efficacy.
2) The drug component is stable, and it is not easy to deteriorate even after being placed for a long time, and it is convenient to store, and it is very suitable for being made into a commercially available drug.
3) The drug is less irritating to the eye and patient compliance is good.
4) It significantly increases the concentration of the active ingredient (can be increased to mM level), so that the concentration meets the requirements of clinical ocular administration.
5) There is no need for surgery or intravitreal injection of drugs for the treatment of cataracts.
6) Econazole is an FDA approved molecular molecule, so that the discovery of this use can quickly enter clinical phase II experiment, which will help shorten the development time and reduce the research and development cost.

The present invention will be further illustrated below with reference to the specific examples. It should be understood that these examples are only to illustrate the invention, not to limit the scope of the invention. The experimental methods with no specific conditions described in the following examples are generally performed under the conventional conditions (e.g., the conditions described by Sambrook et al., Molecular Cloning Laboratory Guide (New York: Cold Spring Harbor Laboratory Press,1989), or according to the manufacture's instructions. Unless indicated otherwise, all percentage and parts are calculated by weight.

### Example 1

### Preparing eye drops:

| | |
|---|---|
| Econazole | 0.0003% |
| Chitosan | 1% |
| Hydroxypropyl-β-cyclodextrin | 40% |

Making up to 100mL with PBS solution

Note: During the preparation process, physical solubilization methods such as conventional ultrasonic, heating, etc. can be used.

The above 7 µM econazole eye drops were administered three times a day including morning, noon, and evening, at intervals of at least 5 hours. The subject of administration was cataract dogs caused by trauma. A total of 9 cases of cataract suffering eye were involved in the test. The mode of administration was directly dropping to the eye to ensure that the drug was completely dripped into the eye. Each case of suffering eye receiving treatment was administrated one drop at a time with an amount of about 50µL, and was continuously administrated for 12 weeks. All 9 cases of cataract suffering eye were observed and judged for signs of symptom ease or symptom relief.

### Example 2

The ophthalmic solution of the present invention was uniform, non-suspension, white liquid, completely aqueous phase, and the co-solvent was cyclodextrin, preferably hydroxypropyl-β-cyclodextrin. The concentration of econazole was 7 µM, and no white particles insoluble in the aqueous phase were observed inside the entire eye drop.

### Eye drops recipe:

| | |
|---|---|
| Econazole | 0.0003% |
| Lanosterol | 1.1364% |
| Chitosan | 1% |
| Hydroxypropyl-β-cyclodextrin | 40% |

Making up to 100mL with PBS solution

Note: During the preparation process, physical solubilization methods such as conventional ultrasonic, heating, etc. can be used.

### Mode of administration:

The above pharmaceutical preparations were administered three times a day including morning, noon, and evening, at intervals of at least 5 hours. The subject of administration was cataract dogs caused by various reasons. The mode of administration was directly dropping to the eye to ensure that the drug was completely dripped into the eye. Each dog receiving treatment was administrated one drop per suffering eye at a time with an amount of about 50µL, and was continuously administrated for 8 weeks. No statin molecule was taken during administration.

### Evaluation indicators:

The opacity of the lens was observed using a slit lamp, and the lens opacity is generally classified into phase 0-V.
phase 0 - Lens are transparent;
phase I- The cortex around the lens is scattered in small vacuoles;
phase II- The cortex around the lens is in ring-shaped dense medium vacuoles;
phase III- Other parts of cortex are in flaky turbidity;
phase IV- Lens nucleus and perinuclear cortex are turbid;
phase V- The lens is completely turbid.

### Therapeutic effect:

The cataract-recovery eye drops prepared according to the present invention can make phase IV or V cataract suffering eyes reduce 2-4 grades after treatment only by the method of administration of eye dropping, without need of surgery or eye intravitreal injection. Especially for traumatic cataract, it can be completely cured after 2 weeks of administration. No dog has discomfort or allergic reaction throughout the treatment.

The above examples show that the addition of polyhydroxy compounds can significantly increase the aqueous phase solubility of the active ingredient in the eye drops. The addition of appropriate thickening agents can increase the stability of the preparation, and promote absorption of the drug during ocular administration, and improve the effect of administration. The ophthalmic preparation provided by the present invention has low irritation and good therapeutic effect, and is particularly suitable for preventing or treating human or non-human mammal cataract.

### Example 3 Econazole eye drops for the treatment of cataract

### Prepare eye drops:

| | |
|---|---|
| Econazole | 0.0003% |
| Tween 80 | 0.1% |
| Hydroxypropyl-β-cyclodextrin | 40% |

Making up to 100mL with PBS solution

Note: During the preparation process, physical solubilization methods such as conventional ultrasonic, heating, etc. can be used.

Rat model of cataract was made: Wistar rats 12 days after birth were selected, male or female, modeled with sodium selenite (senile cataract), each rat was injected subcutaneously in the back neck according to 20 umol/kg body weight. Acupuncture was used to model adult rats (traumatic cataract).

### Evaluation indicators:

The opacity of the lens was observed using a slit lamp, and the lens opacity is generally classified into phase 0-V.
phase 0 - Lens are transparent;
phase I- The cortex around the lens is scattered in small vacuoles;
phase II- The cortex around the lens is in ring-shaped dense medium vacuoles;
phase III- Other parts of cortex are in flaky turbidity;
phase IV- Lens nucleus and perinuclear cortex are turbid;
phase V- The lens is completely turbid.

After 3 days of modeling, econazole eye drops were administered three times a day including morning, noon, and evening, at intervals of at least 5 hours. Every group was made up of 10 cases of cataract suffering eyes involved in the test. The mode of administration was directly dropping to the eye to ensure that the drug was completely dripped into the eye. Each case of suffering eye receiving treatment was administrated one drop at a time with an amount of about 20µL, and was continuously administrated for 10 weeks. All cataract suffering eyes in different treatment groups were observed and judged for signs of symptom ease or symptom relief.

### Therapeutic effect:

The therapeutic effects for cataract are shown in Tables 1 and 2.

**Table 1 treatment effect for nuclear cataract**

| treatment group | phase 0 | phase I | phase II | phase III | phase IV | phase V |
|---|---|---|---|---|---|---|
| econazole | 6 | 0 | 0 | 0 | 4 | 0 |
| PBS group | 0 | 0 | 0 | 0 | 8 | 2 |
| control group | 2 | 0 | 0 | 0 | 6 | 2 |

**Table 2 treatment effect for traumatic cataract**

| treatment group | phase 0 | phase I | phase II | phase III | phase IV | phase V |
|---|---|---|---|---|---|---|
| econazole | 4 | 5 | 1 | 0 | 0 | 0 |
| PBS group | 0 | 0 | 1 | 1 | 6 | 2 |
| control group | 0 | 0 | 1 | 1 | 5 | 3 |

The results show that the cataract-recovery eye drops containing only econazole prepared according to the present invention can treat or prevent cataract very effectively only by the method of eye dropping administration mode, without need of surgery or eye intravitreal injection, especially for traumatic cataracts. No rat has discomfort or ocular allergic reaction throughout the treatment.

### Example 4

### Recipe of eye drop C:

| | |
|---|---|
| Econazole | 0.0003% |
| Lanosterol | 1.1364% |
| Tween 80 | 0.1% |
| Hydroxypropyl-β-cyclodextrin | 40% |

Making up to 100mL with PBS solution

### Recipe of eye drop B:

| | |
|---|---|
| Lanosterol | 1.1364% |
| Tween 80 | 0.1% |
| Hydroxypropyl-β-cyclodextrin | 40% |

Making up to 100mL with PBS solution

### Recipe of eye drop A:

| | |
|---|---|
| Lanosterol | 0% |
| Tween 80 | 0.1% |
| Hydroxypropyl-β-cyclodextrin | 40% |

Making up to 100mL with PBS solution

Note: During the preparation process, physical solubilization methods such as conventional ultrasonic, heating, etc. can be used.

The above-mentioned eye drops of the present invention (recipe B and C) were uniform, non-suspended, white liquid, completely aqueous phase, and the co-solvent was cyclodextrin, preferably hydroxypropyl-β-cyclodextrin, and the concentration of econazole can be 2 µM-40 µM. No white particles insoluble in the aqueous phase were observed inside the entire eye drop.

Cataract rat model aged 30-40 days after birth, which were modeled by using with sodium selenite, were chosen and randomly grouped. After anesthesia and euthanasia, the lenses were carefully removed under the microscope and the cortex was intact. These lenses with the same grade of cataract (phase IV) were randomly grouped and immersed in PBS solvent group (A), a solution containing only lanosterol (recipe B above) or a solution containing both econazole and lanosterol (recipe C above). After placed at room temperature for 7 days in dark, the cortex was removed and changes in the lens nucleus were observed.

The results are shown in Figure 1. The clarity of the lens treated separately with the three recipes was as follows: recipe C was better than recipe B, and recipe B was significantly better than recipe A. It indicated that the solution of group C containing both econazole and lanosterol exhibited the best effect in treatment of nuclear cataract, the eye drops component containing only lanosterol (B) was the second, and the PBS control group (A) had no therapeutic effect on cataract.

The combined results of the above experiments show that the ophthalmic preparation of the present invention (e.g. eye drops) (without lanosterol) has a significant therapeutic effect on cataract in animals. Both senile (nuclear) cataracts and traumatic cataracts can be effectively intervened by the azole compound-containing pharmaceutical preparations of the present invention.

## Claims

1. An ophthalmic preparation for use in the prevention or treatment of cataract, which comprises: (a) a pharmaceutically acceptable carrier, and (b) an econazole compound as a first active ingredient;
wherein the concentration of the econazole compound in the ophthalmic preparation is from 0.005 to 400 µM.

2. The ophthalmic preparation for use according to claim 1, which is selected from the group consisting of: eye drop, an emulsion, gel, eye ointment, sustained-release microsphere, intraocular sustained-release graft, and ocular sustained-release drug film.

3. The ophthalmic preparation for use according to claim 1, which further comprises: (c) a second active ingredient, wherein the second active ingredient is selected from the group consisting of: a steroid compound, glucocorticoid compound, antibiotics, and combinations thereof.

4. The ophthalmic preparation for use according to claim 1, wherein the pharmaceutically acceptable carrier is non-irritating to eye.

5. The ophthalmic preparation for use according to claim 1, which comprises a polyhydroxy compound, wherein the polyhydroxy compound is hydroxypropyl-β-cyclodextrin,
wherein, the content of the polyhydroxy compound is from 0.1 to 50wt%;
based on the total weight of the ophthalmic preparation.

6. The ophthalmic preparation for use according to claim 1, wherein the ophthalmic preparation has a pH value from 5.5 to 8.5

7. The ophthalmic preparation for use according to claim 1, which is an aqueous solution for ocular administration.

8. The ophthalmic preparation for use according to claim 1, which comprises the following ingredients:
0.05-40µM econazole compound;
10-50mM lanosterol compound;
0.1-50wt% polyhydroxy compound, wherein the polyhydroxy compound is hydroxypropyl-β-cyclodextrin;
0-1wt% solubilizer, wherein the solubilizer is polysorbate;
0.2-0.4wt% thickener, wherein the thickener is chitosan;
0-0.5wt% preservative, wherein the preservative is chloramphenicol;
and PBS,
and the ophthalmic preparation has a pH of about 6.5-7.5 and an osmotic pressure of 240-510 mOsm.

9. The ophthalmic preparation for use according to claim 1, wherein said preparation is prepared by a method which comprises the following step:
(1) mixing (a) a pharmaceutically acceptable carrier; and (b) an econazole compound as a first active ingredient, thereby forming the ophthalmic preparation.

10. The ophthalmic preparation for use according to claim 1, wherein the ophthalmic preparation is administered to an eye or two eyes of a subject in need thereof.

## Patentansprüche

1. Ophthalmisches Präparat zur Verwendung bei der Vorbeugung oder Behandlung von grauem Star, das umfasst: (a) einen pharmazeutisch akzeptablen Träger, und (b) eine Econazolverbindung als ersten Wirkstoff;
wobei die Konzentration der Econazolverbindung in dem ophthalmischen Präparat zwischen 0,005 und 400 µM liegt.

2. Ophthalmisches Präparat zur Verwendung gemäß Anspruch 1, welches ausgewählt ist aus der Gruppe bestehend aus: Augentropfen, einer Emulsion, einem Gel, einer Augensalbe, einer Mikrosphäre mit verzögerter Freisetzung, einem intraokularen Transplantat mit verzögerter Freisetzung und einem okularen Wirkstofffilm mit verzögerter Freisetzung.

3. Ophthalmisches Präparat zur Verwendung gemäß Anspruch 1, welches ferner umfasst: (c) einen zweiten Wirkstoff, wobei der zweite aktive Wirkstoff ausgewählt ist aus der Gruppe bestehend aus: einer Steroidverbindung, einer Glucocorticoidverbindung, Antibiotika und Kombinationen davon.

4. Ophthalmisches Präparat zur Verwendung gemäß Anspruch 1, wobei der pharmazeutisch akzeptable Träger für das Auge nicht-reizend ist.

5. Ophthalmisches Präparat zur Verwendung gemäß Anspruch 1, welches eine Polyhydroxyverbindung umfasst, wobei die Polyhydroxyverbindung Hydroxypropyl-β-cyclodextrin ist,
wobei der Anteil der Polyhydroxyverbindung zwischen 0,1 und 50 Gew.-% beträgt, bezogen auf das Gesamtgewicht des ophthalmischen Präparats.

6. Ophthalmisches Präparat zur Verwendung gemäß Anspruch 1, wobei das ophthalmische Präparat einen PH-Wert zwischen 5,5 und 8,5 hat.

7. Ophthalmisches Präparat zur Verwendung gemäß Anspruch 1, das eine wässrige Lösung zur okularen Verabreichung ist.

8. Ophthalmisches Präparat zur Verwendung gemäß Anspruch 1, das die folgenden Inhaltsstoffe umfasst:
0,05-40µM Econazolverbindung;
10-50mM Lanosterolverbindung;
0,1-50 Gew.-% Polyhydroxyverbindung, wobei die Polyhydroxyverbindung Hydroxypropyl-β-cyclodextrin ist;
0-1 Gew.-% Lösungsvermittler, wobei der Lösungsvermittler Polysorbat ist;
0,2-0,4 Gew.-% Verdickungsmittel, wobei das Verdickungsmittel Chitosan ist;
0-0,5 Gew.-% Konservierungsmittel, wobei das Konservierungsmittel Chloramphenicol ist;
und PBS,
und das ophthalmische Präparat einen pH von etwa 6,5-7,5 und einen osmotischen Druck von 240-510 mOsm hat.

9. Ophthalmisches Präparat zur Verwendung gemäß Anspruch 1, wobei das Präparat durch ein Verfahren vorbereitet wird, das den folgenden Schritt umfasst:
(1) Mischen (a) eines pharmazeutisch akzeptablen Trägers; und (b) einer Econazolverbindung als einem ersten Wirkstoff, wodurch das ophthalmische Präparat gebildet wird.

10. Ophthalmisches Präparat zur Verwendung gemäß Anspruch 1, wobei das ophthalmische Präparat einem Auge oder zwei Augen eines Subjekts verabreicht wird, welches es benötigt.

## Revendications

1. Préparation ophtalmique pour une utilisation dans la prévention ou le traitement de la cataracte, qui comprend : (a) un véhicule pharmaceutiquement acceptable, et (b) un composé éconazole en tant que premier principe actif ;
dans laquelle la concentration du composé éconazole dans la préparation ophtalmique est de 0,005 à 400 µM.

2. Préparation ophtalmique pour une utilisation selon la revendication 1, qui est choisie dans le groupe constitué par : des gouttes oculaires, une émulsion, un gel, une pommade oculaire, des microsphères à libération prolongée, un greffon intraoculaire à libération prolongée, et un film de médicament oculaire à libération prolongée.

3. Préparation ophtalmique pour une utilisation selon la revendication 1, qui comprend en outre : (c) un deuxième principe actif, dans laquelle le deuxième principe actif est choisi dans le groupe constitué par : un composé stéroïde, un composé glucocorticoïde, un antibiotique, et leurs combinaisons.

4. Préparation ophtalmique pour une utilisation selon la revendication 1, dans laquelle le véhicule pharmaceutiquement acceptable n'est pas irritant pour les yeux.

5. Préparation ophtalmique pour une utilisation selon la revendication 1, qui comprend un composé polyhydroxylé, dans laquelle le composé polyhydroxylé est l'hydroxypropyl-β-cyclodextrine, dans laquelle la teneur en le composé polyhydroxylé est de 0,1 à 50 % en poids par rapport au poids total de la composition ophtalmique.

6. Préparation ophtalmique pour une utilisation selon la revendication 1, laquelle préparation ophtalmique a un pH de 5,5 à 8,5.

7. Préparation ophtalmique pour une utilisation selon la revendication 1, qui est une solution aqueuse pour administration oculaire.

8. Préparation ophtalmique pour une utilisation selon la revendication 1, qui comprend les ingrédients suivants :
composé éconazole 0,05 à 40 µM ;
composé lanostérol 10 à 50 mM ;
0,1 à 50 % en poids de composé polyhydroxylé, lequel composé polyhydroxylé est l'hydroxypropyl-β-cyclodextrine ;
0 à 1 % en poids de solubilisant, lequel solubilisant est un polysorbate ;
0,2 à 0,4 % en poids d'épaississant, lequel épaississant est le chitosane ;
0 à 0,5 % en poids de conservateur, lequel conservateur est le chloramphénicol ;
et de la STP,
et laquelle préparation ophtalmique a un pH d'environ 6,5 à 7,5 et une pression osmotique de 240 à 510 mOsm.

9. Préparation ophtalmique pour une utilisation selon la revendication 1, laquelle préparation est préparée par un procédé qui comprend l'étape suivante :
(1) mélange (a) d'un véhicule pharmaceutiquement acceptable ; et (b) d'un composé éconazole en tant que premier principe actif, ce qui forme ainsi la préparation ophtalmique.

10. Préparation ophtalmique pour une utilisation selon la revendication 1, laquelle préparation ophtalmique est administrée à un œil ou aux deux yeux d'un sujet en ayant besoin.
